# EUROPEAN PATENT SPECIFICATION

(11) **EP 2 040 763 B1**
(45) Date of publication and mention of the grant of the patent: **25.12.2013**
(21) Application number: 07766275.7
(22) Date of filing: 18.07.2007
(51) Int. Cl.: A61L 15/24, A61L 15/44, A61L 15/46, A61L 15/60

(54) **HYDROGEN PEROXIDE DELIVERY SYSTEM**
SYSTEM ZUR ABGABE VON WASSERSTOFFPEROXID
SYSTÈME DE DISTRIBUTION DE PEROXYDE D'HYDROGÈNE

(30) Priority: 19.07.2006 GB 0614278
(43) Date of publication of application: 01.04.2009
(60) Divisional application: 11164916.6
(73) Proprietor: Archimed LLP, Bedfordshire MK44 1LQ (GB)
(72) Inventor: DAVIS, Paul, James, Felmersham Bedfordshire MK43 7EX (GB); AUSTIN, Andrew, John, Wellingborough NN8 2JQ (GB)
(74) Representative: Willett, Christopher David
(86) International application number: PCT/GB2007/002706
(87) International publication number: WO 2008/009925

(56) References cited:
- WO-A-2004/108176
- WO-A-2005/072784
- WO-A-2007/134304
- US-A- 5 792 090

## Description

### Field of the Invention

This invention relates to a delivery system, e.g. a dressing, comprising hydrogen peroxide for application to a part of the human or animal body especially for treatment of skin, e.g. a wound site.

### Background to the Invention

Hydrogen peroxide (H₂O₂) is a known antimicrobial substance for use on the skin and in wounds. It is produced naturally in the body by white blood cells as part of the immune defence activities in response to infection and through the action of the enzyme superoxide dismutase. There are no known microbial evasion mechanisms by which microbes can escape its effects and it has a short lifetime, very rapidly breaking down to water and oxygen in the tissues. However, excessive hydrogen peroxide can be toxic to tissue cells, and the prevailing attitude in the medical community is that its potential toxicity is too great to justify its regular application to skin or open wounds. Even so, very carefully limited doses of hydrogen peroxide can be used as a means to enrich the wound environment with abundant oxygen, provided that little, if any, intact hydrogen peroxide reaches the living tissues. Catalase and other substances that break down hydrogen peroxide are present in the wound, and in the epidermis in sufficient quantities to ensure very rapid decomposition.

For these reasons, there is a need to provide a means for delivering hydrogen peroxide to wounds or skin, that goes beyond the direct application of a source of hydrogen peroxide as a liquid or a film or other format, straight onto wounds or skin. A more controlled method of application is needed, if hydrogen peroxide is to be used effectively for the treatment of wounds or skin.

In particular, if dose levels could be controlled reliably, and automatically, through a simple, practicable mechanism, hydrogen peroxide could be used safely to great advantage. Any such mechanism would have to limit the rate of delivery of hydrogen peroxide to wounds or skin, such that it is instantly and completely broken down to water and oxygen before appreciable quantities can leave the dressing. In this situation, it would be possible to achieve oxygenation, without the tissues of the wound ever experiencing hydrogen peroxide exposure. In certain types of wound dressing or apparatus, hydrogen peroxide can be used to drive the production of iodine from iodide, provided that there is a reactor compartment within the dressing in which the reaction can take place, and that the ingress of hydrogen peroxide can be regulated appropriately. Thus, hydrogen peroxide can be utilised in the treatment of wounds or skin conditions as an antimicrobial agent in its own right, as a means to provide oxygen or as a means to drive iodine production *in-situ* for controlled iodine delivery. It may be desirable for all three functions to be provided simultaneously.

Hydrogen peroxide has been used for many years as an anti-microbial substance for cleansing wounds of all kinds and as a biologically compatible general antiseptic. It is often used for household and surface cleaning as a more environmentally acceptable and safer alternative to "bleach" (solutions of sodium hypochlorite). In medical applications, hydrogen peroxide-containing ointments have been used, e.g., for treatment of leg ulcers, pressure sores, minor wounds and infection. There are, however, problems associated with hydrogen peroxide, as currently used. Hydrogen peroxide solution is unstable, being readily oxidised to water and oxygen; further, hydrogen peroxide at high concentration can be damaging to normal skin and to cells responsible for healing in the wound bed. It is very difficult or even impossible to use hydrogen peroxide as part of a pre-dosed wound dressing, as its instability would make for a product with an inconveniently short shelf-life. The dosing of simple solutions of hydrogen peroxide at the point of application would not provide a sustained delivery over a usefully prolonged period. When it is used in wound treatment (as described in the British Pharmacopoeia, for example) very high concentrations (typically 3%) are needed to achieve a powerful antimicrobial effect over a very short time interval. Even this type of short burst can be effective, because of the antimicrobial effectiveness of hydrogen peroxide and the physical cleaning effect of the inevitable foaming that occurs as copious amounts of gaseous oxygen are released, but there is the further disadvantage that such high concentrations can be relatively damaging to host cells and can impede the healing process. For this reason, use of hydrogen peroxide tends to be restricted to initial clean-up and sterilisation of wounds. Even so, it is a natural defence substance, produced by the body's own cells (albeit at lower concentrations) and it is increasingly recognised as an intercellular and intracellular messenger molecule, involved in cell to cell molecular signalling and regulation. Undoubtedly, hydrogen peroxide is potentially a very beneficial molecule, if it can be used at the right concentrations over an appropriate time course and with the right accessory molecules or formulations.

WO2005/072784 concerns a skin dressing comprising a hydrated hydrogel material including a source of lactate ions and a supply of glucose, which may be used in conjunction with a superposed layer containing a supply of pre-formed hydrogen peroxide or a hydrogen peroxide precursor substance.

### Summary of the Invention

The invention provides a hydrogen peroxide delivery system, which is free of a source of lactate ions and a supply of glucose, and for delivering hydrogen peroxide to a part of a human or animal body, comprising, prior to use, a component comprising hydrogen peroxide, and a component in hydrated condition, such that, in use, when the components are placed in contact with each other, and arranged with the hydrated component nearer the skin than the hydrogen peroxide component, hydrogen peroxide migrates towards the hydrated component.

The upper component may be in wet or dry condition, but is preferably in dry condition for reasons of storage stability.

The delivery system is suitable for delivery of hydrogen peroxide to a part of the human or animal body. However, typically it will be applied to the skin, e.g. a wound site, and in this case the delivery system takes the form of a dressing and the upper and lower components are dressing components.

The delivery system is designed to be used as a single unit, wherein the two components are brought together at the point of use. The lower component means the component which is nearer the skin in use, with the upper component being located on top of the lower dressing component.

The components are constructed of a material that can be dispensed as a coherent entity, whether in sheet (or film) form, or as an amorphous gel (e.g. that can be squeezed from a dispenser) and which will stay in place when applied to a target site (e.g. a wound or an area of skin.

'Dry condition' means that there is no free water in the material, such that no significant or measurable water loss occurs through evaporation under normal ambient conditions of temperature, pressure and humidity. Dry condition includes desiccated condition, which is an extra thoroughly dried condition. Desiccated condition means a condition maintained by storage in an environment enclosed by a moisture impermeable barrier, wherein the material is kept scrupulously free of water by means of an added desiccant.

In embodiments in which the upper component is in dry condition the hydrogen peroxide is particularly stable and is retained in the material. The upper component can be stored under suitable conditions for an extended period of time, with the hydrogen peroxide remaining stable therein.

### Upper hydrogen peroxide-containing component

The hydrogen peroxide is incorporated in the upper component, which can be considered to be a "carrier" material for the hydrogen peroxide. The upper component may be in a dried form, if that is more convenient or cost effective, but it is equally acceptable for the carrier material to be in a hydrated condition, such as a high water hydrogel. The hydrogen peroxide is preferably dispersed throughout the upper component. Typically the upper component comprises a matrix (whether in dry or hydrated condition) with the hydrogen peroxide dispersed therein, preferably in a reasonably homogeneous manner.

The upper component preferably comprises a polymer material.

A preferred polymer material comprises polyvinyl alcohol (PVA). PVA has convenient and acceptable properties for skin treatment use, e.g. being non-toxic. PVA is also easy to handle and use, readily forming a film on drying of a PVA solution in water, with the resulting film being easy to handle. PVA is also readily available and cheap. Cross-linking is not required to form a solid material, e.g. in the form of a film, although cross-linking may optionally be employed. PVA is available in a wide range of grades based on molecular weight and degree of hydrolysis, which affect the physical properties of the material. Appropriate grades of PVA can be readily selected to produce a polymer product having desired properties for a particular intended use. For example, for use in skin dressings, good results have been obtained by use of PVA with a molecular weight in the range 100,000 to 200,000, substantially fully hydrolysed (98-99% hydrolysed), e.g. in the form of code 36, 316-2 from Aldrich, in non-cross-linked form.

Another preferred polymer material comprises polyvinylpyrrolidone (PVP). The properties of PVP are very similar to those of PVA, and PVP is also acceptable for skin treatment use. PVP is readily available in a range of different molecular weights. Appropriate grades of PVP can be readily selected. For example, good results have been obtained using a PVP having a molecular weight average of 360,000, e.g. in the form of code PVP360 from Sigma, in a non-crosslinked form.

Mixtures of polymer materials may be used, with the presence of at least some PVP being found to be beneficial for hydrogen peroxide stability.

The form of the upper component may be selected to suit the intended use. For use in skin dressings, the component is conveniently in the form of a sheet, layer or film. The layer or film typically has a thickness in the range 0.01 to 1.0mm, preferably in the range 0.05 to 0.5mm.

The hydrogen peroxide may be incorporated in the upper component in the form of hydrogen peroxide *per se* or hydrogen peroxide in combination with or complexed with another entity. Good results have been obtained with a hydrogen peroxide urea complex: this is available as a dry powder, and so is easy to handle, yet will release hydrogen peroxide.

The hydrogen peroxide material may optionally include a support to provide rigidity when wet.

The hydrogen peroxide material may also include a humectant, e.g. glycerol or propylene glycol, to aid in the flexibility of the dried film.

The upper component is conveniently made by mixing a solution of a polymer (e.g. an aqueous solution of PVA and/or PVP) and hydrogen peroxide (e.g. an aqueous solution of hydrogen peroxide urea complex), and drying the mixture to produce a solid material, e.g. forming film by a casting procedure. Suitable techniques are well known to those skilled in the art.

### Lower hydrated component

The lower component is in a hydrated condition, which means that it contains sufficient water for the hydrogen peroxide carried in the upper component to diffuse through its structure and to the interface with the target, e.g. wound or skin. If the upper component is supplied in a dry condition, it will become hydrated (wetted) by contact with the water of the lower component. In this instance, the hydrogen peroxide will be dissolved and released into the lower component. Sufficient water is required within the lower component to form a contact liquid junction between the material and a water source.

Additionally, the lower component provides a source of moisture which can act in use to maintain a beneficial moist environment within a target wound site.

The material of the lower component may be in the form of hydrogel, a sponge, a foam or some other form of hydrophilic matrix that can hold sufficient water to allow a controlled diffusion path between the hydrogen peroxide layer and the target site. Preferably, the water will contain solutes that serve to regulate the passage of hydrogen peroxide, e.g. by hydrogen bonding, which may be achieved by appropriate concentrations of polymers, e.g. polysaccharides, including glycosaminoglycans. Preferably the layer will contain polyacrylic polymers, such as poly 2-acrylamido-2-methylpropane sulphonic acid (polyAMPS), which serve to impart solid gel properties as well as the ability to control transmission of hydrogen peroxide.

The lower component can control hydrogen peroxide flux rates in numerous ways, including by selection of its physical dimensions (especially depth, affecting diffusion path distance), its water content (less water causing a slower diffusion rate), its composition (with immobilised hydrogen bonding groups slowing hydrogen peroxide movement) and/or its surface architecture at the interface with the target site, e.g. wound site, and/or at the interface with the upper component (affecting the contact surface areas and thereby the rate of transfer into or out of the lower component), e.g., it may have a contoured (possibly corrugated) surface.

Because the upper component releases hydrogen peroxide when wet, the water-bearing lower component can serve as both a source of water for re-hydration/dissolution of the hydrogen peroxide (if in dry condition) and as the means for the hydrogen peroxide to pass through the delivery system, e.g. for delivery to a wound from a dressing. The rate of hydration/dissolution can be controlled by the combined properties of the two layers, and the way they interact. This, in turn, can regulate the rate at which hydrogen peroxide is made available to e.g. a wound, and can be used to ensure that the dosage is sufficient only to effectively expose a wound to oxygen, rather than to hydrogen peroxide (through the effect of tissue catalase and other hydrogen peroxide-decomposing substances in immediate contact with the dressing).

In addition, the lower component can perform as a reactor, in which the hydrogen peroxide is actively decomposed to oxygen and water (e.g. by containing iodide ions, which undergo a complex chemical reaction with the hydrogen peroxide, resulting in appropriate oxygen production). The lower component can also provide the benefit of synthesising and delivering active wound-care substances, typically by means of chemical reaction with hydrogen peroxide. For example, iodine can be synthesised in this way, through the oxidation of iodide ions, to work as an antimicrobial agent. As with hydrogen peroxide, it is helpful for the wound to receive iodine at a controlled rate such that there is sufficient iodine to kill microbes, but the level is low enough to avoid toxic effects on the wound tissues. Another example is provided by the delivery of allantoin, which is claimed to have a healing effect. Allantoin is unstable, so it is preferable for the transmission layer to be pre-dosed with relatively stable urate ions, which are oxidised by incoming hydrogen peroxide to yield allantoin.

Typically, skin or a wound is in direct contact with the water-bearing lower component. The lower component, preferably when in the form of a hydrated hydrogel as discussed below, can (depending on its chemical composition) act to absorb water and other materials exuded from a wound site, enabling the dressing to perform a valuable and useful function by removing such materials from a wound site.

The form of the lower component may be selected to suit the intended use. For use in skin dressings, the material is conveniently in the form of a sheet, layer or film. The layer or film typically has a thickness in the range 0.01 to 1.0mm, preferably in the range 0.05 to 0.5mm.

The lower component may alternatively be in the form of an amorphous gel or lotion, preferably a hydrogel, not having a fixed form or shape, that can be deformed and shaped in three dimensions, including being squeezed through a nozzle. Amorphous gels are typically not cross-linked or have low levels of cross-linking. A shear-thinning amorphous gel may be used. Such a gel is liquid when subjected to shear stress (e.g. when being poured or squeezed through a nozzle) but is set when static. Thus the gel may be in the form of a pourable or squeezable component that may be dispensed, e.g. from a compressible tube or a syringe-like dispenser, comprising a piston and cylinder, typically with a nozzle of about 3mm diameter. Such a gel may be applied in the form of a surface layer, or into a wound cavity as a fully conformable gel that fills the available space and contacts the wound surface.

This approach finds particular application in the treatment of cavity wounds by, for example, squeezing from a tube or syringe, with the cavity being filled with the amorphous gel and an upper component (e.g. a film, possibly in rolled up condition) placed onto the gel. It is also possible for the material to be carried in the form of rope or tape to be packed into a cavity. On wetting of the upper component, e.g. by water from the gel or lotion, hydrogen peroxide is released and reacts with catalase of the wound site to produce oxygen. If the gel or lotion includes iodide ions then either predominantly oxygen will be produced within the gel (if the iodide ions are at a low concentration) or substantial levels of iodine and oxygen will be generated (if the iodide ions are at a suitably increased concentration).

A typical example of an amorphous gel formulation is: 15%w/w AMPS (sodium salt), 5 % w/w glucose, 0.05% w/w potassium iodide, 0.1% zinc lactate, 0.19% polyethylene glycol diacrylate and 0.01% hydroxycyclohexyl phenyl ketone, with the volume made up to 100 % with analytical grade DI water. The reagents are thoroughly mixed and dissolved, then polymerised for between 30-60 seconds, using a UV-A lamp delivering approximately 100mW/cm², to form the required hydrogel. This may be in the form of a flat sheet or, more conveniently, housed in plastic syringes. The amorphous gel may then be dispensed from a syringe into a target site.

### Hydrogels

The lower component is preferably in the form of a hydrated hydrogel. A hydrated hydrogel means one or more water-based or aqueous gels, in hydrated form. A hydrated hydrogel can act to absorb water and other materials exuded from a wound site, enabling the dressing to perform a valuable and useful function by removing such materials from a wound site. The hydrated hydrogel also provides a source of moisture, that can act in use to maintain a wound site moist, aiding healing. The hydrated hydrogel also acts as a source of water, causing release of hydrogen peroxide. Use of a hydrated hydrogel has other benefits as discussed in WO 03/090800.

Suitable hydrated hydrogels are disclosed in WO 03/090800. The hydrated hydrogel conveniently comprises hydrophilic polymer material. Suitable hydrophilic polymer materials include polyacrylates and methacrylates, e.g. as supplied by First Water Ltd in the form of proprietory sheet hydrogels, including poly 2-acrylamido-2-methylpropane sulphonic acid (polyAMPS) or salts thereof (e.g. as described in WO 01/96422), polysaccharides e.g. polysaccharide gums particularly xanthan gum (e.g. available under the Trade Mark Keltrol), various sugars, polycarboxylic acids (e.g. available under the Trade Mark Gantrez AN-169 BF from ISP Europe), poly(methyl vinyl ether co-maleic anhydride) (e.g. available under the Trade Mark Gantrez AN 139, having a molecular weight in the range 20,000 to 40,000), polyvinyl pyrrolidone (e.g. in the form of commercially available grades known as PVP K-30 and PVP K-90), polyethylene oxide (e.g. available under the Trade Mark Polyox WSR-301), polyvinyl alcohol (e.g. available under the Trade Mark Elvanol), cross-linked polyacrylic polymer (e.g. available under the Trade Mark Carbopol EZ-1), celluloses and modified celluloses including hydroxypropyl cellulose (e.g. available under the Trade Mark Klucel EEF), sodium carboxymethyl cellulose (e.g. available under the Trade Mark Cellulose Gum 7LF) and hydroxyethyl cellulose (e.g. available under the Trade Mark Natrosol 250 LR).

Mixtures of hydrophilic polymer materials may be used in a gel.

In a hydrated hydrogel of hydrophilic polymer material, the hydrophilic polymer material is desirably present at a concentration of at least 1%, preferably at least 2 % , more preferably at least 5%, yet more preferably at least 10 % , or at least 20 % , desirably at least 25 % and even more desirably at least 30 % by weight based on the total weight of the gel. Even higher amounts, up to about 40 % by weight based on the total weight of the gel, may be used.

A preferred hydrated hydrogel comprises poly 2-acrylamido-2-methylpropane sulphonic acid (poly AMPS) or salts thereof, preferably in an amount of about 30 % by weight of the total weight of the gel.

The lower component can be manufactured by known means. Preferably it is manufactured by the polymerisation of AMPS monomer dissolved at the rate of about 40 % w/v in a solution buffered to a pH of about 5.5, containing any further ingredients required for controlling the rate of hydrogen peroxide transmission or reaction, such as iodide. If iodide is required primarily only to decompose hydrogen peroxide to oxygen and water, the iodide concentration should be about 0.01% w/v. If it is to be used both to release oxygen and synthesise iodine, then the level of iodide should be from about 0.05% to about 0.2% w/v. Methods for the manufacture of this material are as described in patent number EP1631328.

### Use

For use on the body, the delivery system, e.g. a skin dressing, may be assembled simply by laying the upper component onto the lower component. This can be carried out away from the skin/wound surface, in which case the composite dressing is placed on the skin/wound as a single entity, with the hydrogen peroxide upper component facing outwards, away from the skin surface. Alternatively, the lower component can be applied first to the target skin or wound site, and then the hydrogen peroxide component can be added on top. Both components may also be cut to size, should the dressing be too large for the area to be treated, where the upper component remains smaller than the lower component, thus preventing the upper component from coming in contact with the skin or wound surface directly.

Usually the lower component will be located directly on the body, but it is possible for intervening material to be present.

For most body treatments, the delivery system is used for skin treatment by being located on or near the skin of a human or animal, e.g. over a wound or on a region of skin to be treated for cosmetic or therapeutic purposes, e.g. for treatment of a wide range of conditions as discussed above.

If the upper component is in a dried condition (e.g. a PVA film), it will be automatically supplied with water from the lower component, as soon as they are brought together at the point of use. Once rehydrated, the hydrogen peroxide can migrate into the lower component and thence to the interface between the dressing and the wound or skin at a pre-determined rate. If the hydrogen peroxide layer is already hydrated (e.g. a 40% w/v polyAMPS hydrogel with dissolved hydrogen peroxide) then it just remains for the hydrogen peroxide to diffuse into and through the lower component. In both situations, hydrogen peroxide is released at an appropriate rate, with known beneficial effects as discussed above.

In particular, the composite dressing may be used in a skin treatment dressing or wound dressing.

### Optional ingredients

In addition to components essential for controlling the passage of hydrogen peroxide and/or reacting with the hydrogen peroxide to generate benefit agents in place, the dressing, and preferably the lower component, may incorporate one or more other active ingredients such as zinc ions, as disclosed in WO 2004/108917. Zinc ions are known to form stabilising complexes with hydrogen peroxide, aiding delivery of hydrogen peroxide to the target site. Zinc is also an essential, nutritional trace element, which has numerous functions in the growth and repair of healthy tissues.

### Packaging

The delivery system conveniently includes, or is used with, a covering or outer layer for adhering the dressing to the skin of a human or animal subject in known manner.

The components of the delivery system are preferably separately packaged for optimal performance prior to use, e.g. being sealed in suitable sterile water-impervious packages, e.g. of laminated aluminium foil.

### Preferred embodiments

In a preferred embodiment the delivery system is a dressing which comprises an upper component in dry condition, in the form of a layer, comprising a hydrogen peroxide urea complex and PVA, and a lower component which is a poly-AMPS hydrogel in the form of a layer.

The invention will be further described, by way of illustration, in the following examples which refer to the accompanying drawings, in which:
Figure 1 is a graph of current (in micro Amps) versus time (in mins) showing iodine production from a polymer film in accordance with the invention in combination with an iodine-containing hydrogel transmission layer;
Figure 2 is a graph of an oxygenation effect (compared to atmospheric oxygen at 100%) versus time (in mins) showing oxygen production from a polymer film in accordance with the invention in combination with a hydrogel layer; and
Figure 3 is a graph of H₂O₂ recovery from dry stored films, expressed as µg H₂O₂ recovered per milligram of film, versus storage time (in days).
Figure 4 is a graph of current (in micro Amps) versus time (in mins) showing iodine production from a polymer film in accordance with the invention and from polymer films comprising glucose and/or lactate ions.

### Example 1

Polyvinyl alcohol (PVA) (98-99% hydrolysed, 124,000-186,000 molecular weight, code 36,316-2 from Aldrich) was dissolved in de-ionised water to a final concentration of 5% w/w. The water was heated to boiling point, and the PVA granules were slowly added, with constant agitation. The water temperature was maintained at 80°C or above, until the PVA had dissolved. The PVA solution was allowed to cool to room temperature about (21°C) before use.

Urea-Hydrogen Peroxide (UHP) (containing 35% hydrogen peroxide, code U1753 from Sigma) was added to the 5% PVA solution, to give 1.0% w/w. This gave a final PVA concentration 4.95% w/w. The UHP was readily soluble, and only slight agitation at RT (21°C) was required to dissolve the powder.

To form the dry films, constituting an upper component, a plastic container with a surface area of 124cm² was used. Into this, either 10 or 20 grams of the UHP/PVA solution was poured. The UHP/PVA solutions were spread evenly over the entire surface, and placed at 40°C for 16-24 hours to dry. After the films were dried, they were removed and kept in air tight polythene bags, at RT (21°C). The films had a thickness of about 0.1mm or 0.2mm, depending on the amount of UHP/PVA solution used.

To assess the release of hydrogen peroxide (H₂O₂) from the UHP/PVA films, the production of iodine and oxygen were measured from a secondary hydrated hydrogel using amperometric electrochemistry.

A hydrated hydrogel layer, constituting a lower component, was formulated to include the following ingredients by weight:

| | |
|---|---|
| Water (ex Fisher, distilled, de-ionised, analytical grade) | 64.7% |
| Sodium AMPS (ex Lubrizol AMPS 2405 Monomer) | 30.0% |
| Polyethylene glycol diacrylate (PEG400 diacrylate, ex UCB Chemicals, available as Ebecryl 11) (a cross-linker) | 0.19% |
| 1-hydroxycyclohexyl phenyl ketone (ex Aldrich - 40,561-2) (a photoinitiator) | 0.01% |
| Anhydrous glucose, (ex Fisher, analytical grade, code GO50061) | 5.00% |
| Potassium iodide (ex Fisher, analyical grade, P584050) | 0.05% |
| Zinc L-lactate hydrate (ex Aldrich) | 0.10% |

The mixture was dispensed into casting trays containing a polyester scrim (polyester nonwoven, open mesh support, available from HDK Industries Inc, Product Code 5722) of dimensions 100mm x 100mm, to a depth of about 1.5mm. The hydrogel was then set, by exposure to irradiation under a UV lamp for up to 60 seconds at a power rating of approximately 100mW/cm². The hydrogel was then allowed to cool to 30°C or below.

Measurements were made using an Ezescan instrument and software supplied by Whistonbrook Technologies, Luton, UK (Ezescan is a Trade Mark). Measurements were made using a sensor comprising an alumina substrate screen printed with carbon paste (ED5000 from Electra Ltd, UK) to produce 3 electrodes (working, reference and counter electrodes). The reference electrode was further coated with Ag/AgCl paste. To measure oxygen, and to prevent interference from the hydrogen peroxide, the oxygen sensor was wrapped and sealed in a single layer of Teflon fluorocarbon 0.005 inches (0.013mm) thick (Teflon is a Trade Mark). This formed a chamber into which electrode buffer could be placed. To measure iodine production, a potential of -100mV was applied over 16 hours, and to measure oxygen production, a potential of -550mV was applied over 16 hours.

### Iodine Measurement:

The open sensor was attached to the Ezescan instrument via a suitable connector block and lead. The block and sensor were contained inside a chamber, to minimise water loss through evaporation. 25µl of a 0.1M KCl solution was added to the working electrode. A 5 x 5 x 0.1cm square of the secondary hydrogel was placed onto the KCl and sensor, so that the working electrode was under the centre of the hydrogel. A 2 x 2 cm square of the UHP/PVA film was placed onto the centre of the hydrogel, directly above the electrode. The potential was then applied, and the experiment carried out over 16 hours, at 25°C, reading the current generated every minute.

As UHP is released, the peroxide oxidises iodide to iodine and diffuses throughout the hydrogel. The iodine can then be reduced at the electrode, and the current generated used as a marker for the release of peroxide.

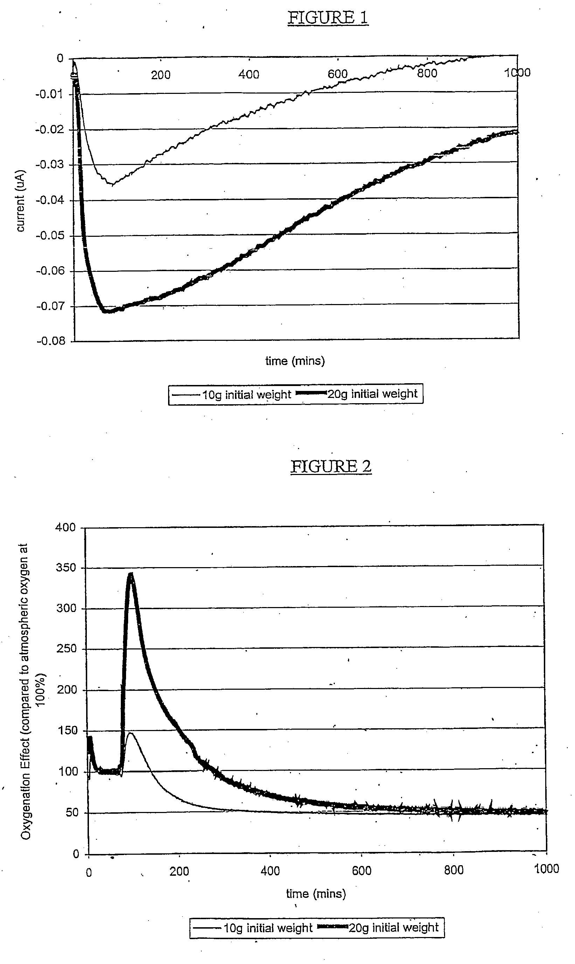

The results are shown in Figure 1. The results show that (i) iodine was produced, and (ii) the different weight of the PVA/UHP film released an increased amount of UHP.

### Oxygen Measurement:

The sensor was filled with 0.1M KCl, and soaked in this solution for 24 hours before use. The sensor was rinsed and refilled with fresh 0.1M KCl. The open end was sealed off, to prevent fluid loss. The sensor was attached to the Ezescan instrument via a suitable connector block and lead. The block and sensor were contained in a chamber, to minimise water loss through evaporation. To the Teflon above the sensor, 25µl de-ionised water was added, and a potential of -550mV then applied. The current response was monitored until this formed a plateau at approximately -2.5µA indicating equilibration with atmospheric oxygen. The water was then removed and replaced with 25µl 0.1mg/ml catalase in water (equivalent to 6 units of activity).

Onto this, a 5 x 5 cm square of the secondary hydrogel was placed, followed by a 2 x 2 cm square of the UHP/PVA film. The experiment was performed over 16 hours, at 25°C, reading the current every minute. The principle of the technique was identical to that of the commercially available 'Clark oxygen sensors'. If oxygen is produced by catalase- mediated decomposition of hydrogen peroxide, it will diffuse through the Teflon layer into the electrode electrolyte and equilibrate in the KCI, where it will be reduced at a working electrode poised at -550 mV vs. the Ag-AgCl reference electrode. The resulting cathodic current is proportional to concentration of dissolved oxygen.

Results are shown in Figure 2, which is a graph of oxygen production expressed as a percentage of atmospheric oxygen (taken as 100%) versus time. The Teflon coated sensor was equilibrated with atmospheric oxygen (the plateau marked as 100%). At approximately 50mins after the start of the run, catalase was applied to the Teflon surface, followed by the hydrogel layer (the same composition as used for the iodine experiments), then the PVA/UHP film was added uppermost.

Figure 2 shows that (i) oxygen was produced and was measurable at the electrode, and (ii) the increased weight of the PVA/UHP films delivered different volumes of oxygen.

### Example 2

5 % w/w PVA solution was prepared as described in Example 1.

5 % w/w PVP solution was prepared by dissolving 5g PVP (360,000 average molecular weight, Sigma Code PVP360) in 95g DI water. The PVP is cold water soluble and does not require any further treatment.

Using these stock solutions, the following were prepared:
Sample 1: to 5% PVP solution, water was added to give 0.5% w/w. Final [PVP] = 4.92% w/w.
Sample 2: to 5% PVA solution, UHP was added to give 1.4% w/w. Final [PVA] = 4.93% w/w. pH = 5.9.
Sample 3: as sample 2, but pH adjusted with small volume of citric acid to give pH 4.3.
Sample 4: to 5% PVA solution, UHP was added to give 1.4% w/w, and PVP was added to give 1% w/w. Final [PVA] = 4.88%. pH = 5.9.
Sample 5: as sample 4, but pH adjusted with small volume of citric acid to give pH 4.3.

10g of each sample was dispensed into an 8.4cm diameter petri dish, and dried at 40°C for 18hours. Samples 2-5 were then stored in a desiccator at RT (about 21°C), while sample 1 was stored undesiccated at RT (about 21°C).

Samples were tested for hydrogen peroxide, using the following method.

10mg of each film was removed and placed into a 7ml bijou container. 1ml of DI water was added, and the samples were soaked for 30mins to allow the hydrogen peroxide to diffuse out. To a 4ml cuvette, 2.2ml DI water, 0.5ml 0.1M Na phosphate pH 5.0 (with citric acid), 0.1ml 1mg/ml lactoperoxidase, 0.1ml 3mg/ml TMB (tetra methyl benzidine) in DMSO were added. 0.5ml of the sample-soaked water was then added to the cuvette, mixed and allowed to stand for 5mins for the colour to develop. After 5mins, the colour was then read at 630nm. The quantity of hydrogen peroxide present was then estimated from a standard curve of hydrogen peroxide in water, assayed using the same assay method.

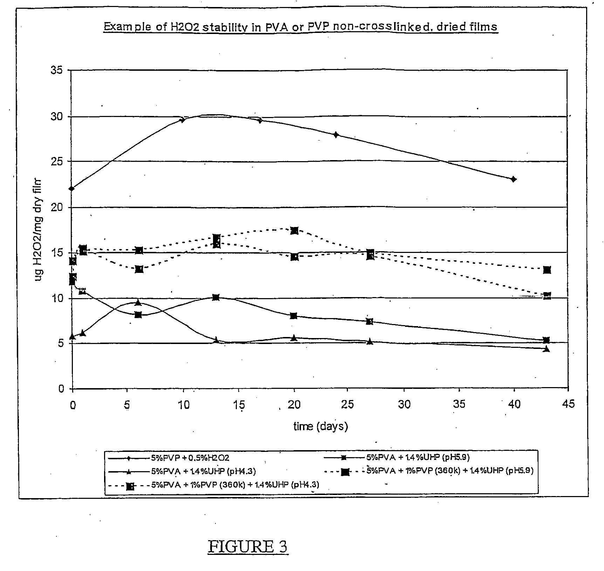

The results in Figure 3 demonstrate the hydrogen peroxide stability in PVP, PVA and PVA+PVP films. Within the testing period, stable hydrogen peroxide films were maintained. The use of PVP would appear to aid hydrogen peroxide stability within the films. This is thought to be due to the known complexation between PVP and hydrogen peroxide.

### Example 3:

5 % w/w PVA solution was prepared as described in Example 1.

PVP (360,000 average molecular weight, Sigma Code PVP360) and H₂O₂ (30%w/w, Sigma Code H1009) were added to the 5% PVA solution to give final concentrations of 1% and 0.5% respectively. PVA final concentration was 4.85%. 20g of this mix was poured into a 10cm² dish and dried at 40°C for 16 hours.

Secondary hydrogel layers were prepared using the following formulations:

| Reagent | Gel 1 | Gel 2 | Gel 3 | Gel 4 |
|---|---|---|---|---|
| Water (ex Fisher, distilled, de-ionised, analytical grade) | 64.7% | 67.8% | 69.7% | 69.8% |
| Sodium AMPS (ex Lubrizol AMPS 2405 Monomer) | 30.0% | 30.0% | 30.0% | 30.0% |
| Polyethylene glycol diacrylate (PEG700 diacrylate, ex Aldrich-455008) (a cross-linker) | 0.19% | 0.19% | 0.19% | 0.19% |
| 1-hydroxycyclohexyl phenyl ketone (ex Aldrich - 40,561-2) (a photoinitiator) | 0.01% | 0.01% | 0.01% | 0.01% |
| Anhydrous glucose, (ex Fisher, analytical grade, code G050061) | 5.00% | 5.00% | 0% | 0% |
| Potassium iodide (ex Fisher, analyical grade, P584050) | 0.05% | 0.05% | 0.05% | 0.05% |
| Zinc L-lactate hydrate (ex Aldrich) | 0.10% | 0% | 0.10% | 0% |

50g of each of the formulations was poured into a 10cm² dish and polymerised under 100mW/ cm² UV radiation, for 25seconds. The gels were removed and stored at 4°C before use.

The effect of glucose and lactate in the secondary hydrogels was examined using iodine as a marker for H₂O₂ release. The open sensor was attached to the Ezescan instrument via a suitable connector block and lead. The block and sensor were contained inside a chamber, to minimise water loss through evaporation. 30µl of a 0.1M KCl solution was added to the working electrode. A 5 x 5cm square of the secondary hydrogel was placed onto the KCl and sensor, so that the working electrode was under the centre of the hydrogel. A 2 x 2 cm square of the H₂O₂/PVA film was placed onto the centre of the hydrogel, directly above the electrode. The potential was then applied, and the experiment carried out over 16 hours, at 25°C, reading the current generated every minute.

As H₂O₂ is released, the peroxide oxidises iodide to iodine and diffuses throughout the hydrogel. The iodine can then be reduced at the electrode, and the current generated used as a marker for the release of peroxide.

### Discussion:

Referring to Example 1 and Figures 1 and 2, iodine and oxygen production were a direct consequence of peroxide release. When the dried PVA/UHP layer was placed onto the hydrogel, the PVA film hydrated sufficiently to allow the release of the UHP. The PVA film remained intact and could be removed complete. Because the hydrogel contained iodide ions, the peroxide reacted with these to form iodine, following the equation:

H₂O₂ + 2 I⁻ + 2H⁺ → I₂ + 2H₂O

The iodine would then diffuse through the gel and to the sensor electrode. As Figure 1 shows, the response was UHP volume dependant, with the heavier weight of PVA film delivering a larger iodine response. When the iodide was exhausted, the iodine graphs declined. This was due to the iodine in the hydrogel vaporising from the surface, thus driving the iodine concentration in the hydrogel down.

The oxygenation graph of Figure 2 also demonstrated the release of UHP from the PVA film, after hydration in contact with the hydrogel. The sensor was equilibrated with ambient air, to gain a stabilised graph. This was taken as 100%. A decrease from this point would indicate that the oxygen concentration at the sensor was being lowered, while any increase would show that the oxygen concentration was being raised above that of the ambient air. Figure 2 clearly showed that an increase in the oxygen concentration relative to that of air was taking place. Again, the weight of the PVA film gave a different oxygen response, indicating that the increased PVA film thickness was able to deliver more UHP into the hydrogel below. Also, the hydrogel formulation was the same as that used with the iodine experiments, i.e. the hydrogel contained iodide, and so was producing iodine during the oxygenation experiment. This showed that UHP was in excess, and was able to drive both the iodine and oxygen production.

Further, the graph presented in Figure 3 clearly demonstrates that hydrogen peroxide can be formulated as stable, dry films. Hydrogen peroxide was formulated into PVA-only films, PVA + PVP films, and a PVP-only film. PVP is known to form complexes with hydrogen peroxide, and it is therefore assumed that the PVP present in the formulations aided hydrogen peroxide stability during drying and storage. The graph showed that as the % PVP was increased (from 0 % to 1 % to 5%), the recoverable hydrogen peroxide also increased. The use of PVP in the formulation is therefore advantageous as an hydrogen peroxide stabiliser, but it is not essential since PVA-only films also provide a stable hydrogen peroxide recovery, albeit at a lower level.

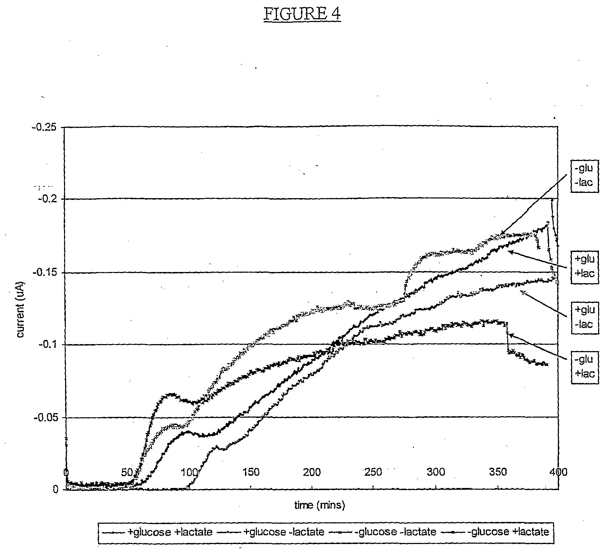

Figure 4 shows iodine production in the presence or absence of glucose and lactate within the hydrogel. Generally, the graphs show that there is no gross effect on the production of iodine, although there are small differences. The most likely cause of these differences is the variation in thickness of the PVA/PVP/H₂O₂ films, caused by the drying process, which would yield different doses of H₂O₂ into the iodide containing hydrogel. But overall, the graph shows that iodine production is not hindered by the absence of glucose, lactate or both, i.e. iodine production using H₂O₂ is not dependant on the presence of glucose and/or lactate in the hydrogel layer.

## Claims

1. A hydrogen peroxide delivery system, which is free of a source of lactate ions and a supply of glucose, and for delivering hydrogen peroxide to a part of a human or animal body, comprising, prior to use, a component comprising hydrogen peroxide, and a component in hydrated condition, such that, in use, when the components are placed in contact with each other, and arranged with the hydrated component nearer the skin than the hydrogen peroxide component, hydrogen peroxide migrates towards the hydrated component.

2. A delivery system according to claim 1, wherein the hydrogen peroxide component is in dry condition prior to use.

3. A delivery system according to claim 1 or claim 2, which is a skin dressing and the two components are dressing components.

4. A delivery system according to any one preceding claim, wherein the hydrogen peroxide component comprises a polymer material.

5. A delivery system according to claim 4, wherein the polymer material comprises polyvinyl alcohol.

6. A delivery system according to any one preceding claim, wherein the hydrogen peroxide component is in the form of a sheet, layer or film.

7. A delivery system according to any one preceding claim, wherein the hydrogen peroxide is in the form of a hydrogen peroxide urea complex.

8. A delivery system according to any one preceding claim, wherein the hydrated component comprises a hydrated hydrogel.

9. A delivery system according to any one preceding claim, wherein the hydrated component comprises iodide ions.

10. A delivery system according to any one preceding claim, wherein the hydrated component is in the form of a sheet, layer or film.

11. A delivery system according to any one of claims 1 to 9, wherein the hydrated component is in the form of an amorphous gel or lotion.

12. A delivery system according to any one preceding claim, which comprises zinc ions.

13. A delivery system according to any one preceding claim, wherein the two components are separately packaged prior to use.

## Patentansprüche

1. Wasserstoffperoxidzuführungssystem, das frei von einer Lactationenquelle und einem Glucosevorrat ist, zur Zuführung von Wasserstoffperoxid zu einem Teil eines menschlichen oder tierischen Körpers, das vor der Verwendung eine Wasserstoffperoxid umfassende Komponente und eine Komponente in hydratisiertem Zustand umfasst, so dass bei der Verwendung bei Anordnung der Komponenten in Kontakt miteinander und mit der hydratisierten Komponente näher zur Haut als die Wasserstoffperoxidkomponente Wasserstoffperoxid zu der hydratisierten Komponente wandert.

2. Zuführungssystem nach Anspruch 1, wobei die Wasserstoffperoxidkomponente vor der Verwendung in trockenem Zustand vorliegt.

3. Zuführungssystem nach Anspruch 1 oder Anspruch 2, bei dem es sich um einen Hautverband handelt und es sich bei den beiden Komponenten um Verbandkomponenten handelt.

4. Zuführungssystem nach einem der vorhergehenden Ansprüche, wobei die Wasserstoffperoxidkomponente ein Polymermaterial umfasst.

5. Zuführungssystem nach Anspruch 4, wobei das Polymermaterial Polyvinylalkohol umfasst.

6. Zuführungssystem nach einem der vorhergehenden Ansprüche, wobei die Wasserstoffperoxidkomponente in Form eines Flächengebildes, einer Schicht oder eines Films vorliegt.

7. Zuführungssystem nach einem der vorhergehenden Ansprüche, wobei das Wasserstoffperoxid in Form eines Wasserstoffperoxid-Harnstoff-Komplexes vorliegt.

8. Zuführungssystem nach einem der vorhergehenden Ansprüche, wobei die hydratisierte Komponente ein hydratisiertes Hydrogel umfasst.

9. Zuführungssystem nach einem der vorhergehenden Ansprüche, wobei die hydratisierte Komponente Iodidionen umfasst.

10. Zuführungssystem nach einem der vorhergehenden Ansprüche, wobei die hydratisierte Komponente in Form eines Flächengebildes, einer Schicht oder eines Films vorliegt.

11. Zuführungssystem nach einem der Ansprüche 1 bis 9, wobei die hydratisierte Komponente in Form eines amorphen Gels oder einer Lotion vorliegt.

12. Zuführungssystem nach einem der vorhergehenden Ansprüche, das Zinkionen umfasst.

13. Zuführungssystem nach einem der vorhergehenden Ansprüche, wobei die beiden Komponenten vor der Verwendung separat verpackt sind.

## Revendications

1. Système de distribution de peroxyde d'hydrogène, qui est exempt d'une source d'ions lactate et d'un apport de glucose, et destiné à distribuer du peroxyde d'hydrogène au niveau d'une partie d'un corps humain ou animal, comprenant, avant l'utilisation, un composant comprenant du peroxyde d'hydrogène et un composant dans un état hydraté, de manière telle que lors de l'utilisation, lorsque les composants sont mis en contact l'un avec l'autre et agencés avec le composant hydraté plus proche de la peau que le composé de type peroxyde d'hydrogène, le peroxyde d'hydrogène migre vers le composant hydraté.

2. Système de distribution selon la revendication 1, le composant de type peroxyde d'hydrogène étant dans un état sec avant l'utilisation.

3. Système de distribution selon la revendication 1 ou la revendication 2, qui est un pansement dermique et les deux composants étant des composants de pansement.

4. Système de distribution selon l'une quelconque revendication précédente, le composant de type peroxyde d'hydrogène comprenant un matériau polymère.

5. Système de distribution selon la revendication 4, le matériau polymère comprenant du poly(alcool vinylique).

6. Système de distribution selon l'une quelconque revendication précédente, le composant de type peroxyde d'hydrogène étant sous forme d'une feuille, d'une couche ou d'un film.

7. Système de distribution selon l'une quelconque revendication précédente, le peroxyde d'hydrogène étant sous forme d'un complexe peroxyde d'hydrogène-urée.

8. Système de distribution selon l'une quelconque revendication précédente, le composant hydraté comprenant un hydrogel hydraté.

9. Système de distribution selon l'une quelconque revendication précédente, le composant hydraté comprenant des ions iodure.

10. Système de distribution selon l'une quelconque revendication précédente, le composant hydraté étant sous forme d'une feuille, d'une couche ou d'un film.

11. Système de distribution selon l'une quelconque des revendications 1 à 9, le composant hydraté étant sous forme d'un gel amorphe ou d'une lotion.

12. Système de distribution selon l'une quelconque revendication précédente, qui comprend des ions de zinc.

13. Système de distribution selon l'une quelconque revendication précédente, les deux composants étant emballés séparément avant l'utilisation.
